# EUROPEAN PATENT SPECIFICATION

(11) **EP 3 930 603 B1**
(45) Date of publication and mention of the grant of the patent: **25.06.2025**
(21) Application number: 20713394.3
(22) Date of filing: 28.02.2020
(51) Int. Cl.: A61B 17/34, A61M 25/06, A61M 25/01, A61B 90/00

(54) **SHEATH ASSEMBLY HAVING AN ECHOGENIC STRUCTURE**
HÜLLENANORDNUNG MIT ECHOGENER STRUKTUR
GAINE AYANT UNE STRUCTURE ÉCHOGÈNE

(30) Priority: 28.02.2019 NL 2022656
(43) Date of publication of application: 05.01.2022
(73) Proprietor: Sono-Coat B.V., 7521 AG Enschede (NL)
(72) Inventor: BREEK, Hendricus Philip, 1217 KZ Hilversum (NL); AYRES, Lee, 6524 SE Nijmegen (NL)
(74) Representative: EP&C
(86) International application number: PCT/NL2020/050131
(87) International publication number: WO 2020/175995

(56) References cited:
- WO-A1-2015/135520
- CN-A- 109 009 359
- US-A- 5 217 438
- US-A- 5 437 645
- US-A1- 2001 021 824
- US-A1- 2012 059 308
- US-A1- 2013 261 650
- US-A1- 2017 112 528
- US-A1- 2017 135 721

## Description

### FIELD OF THE INVENTION

The present invention relates to an improved echogenic sheath assembly.

### BACKGROUND OF THE INVENTION

Sheath assemblies having an echogenic feature are known, for instance from WO2008/098293. See in particular figures 7 - 11B of WO2008/098293 and the description related to these figures.

Such sheath assemblies can be used in conjunction with medical devices, such as needles. The echogenic structure allows a medical practitioner, such as physician or nurse to see the medical device on the screen of an ultrasound machine.

These sheaths may be used for various kinds of needles, for instance; biopsy needles, peripheral nerve block needles, microwave, cryogenic, radiofrequency ablation needles, vascular access needles, infusion needles. Obviously, this is a non-limiting list of examples.

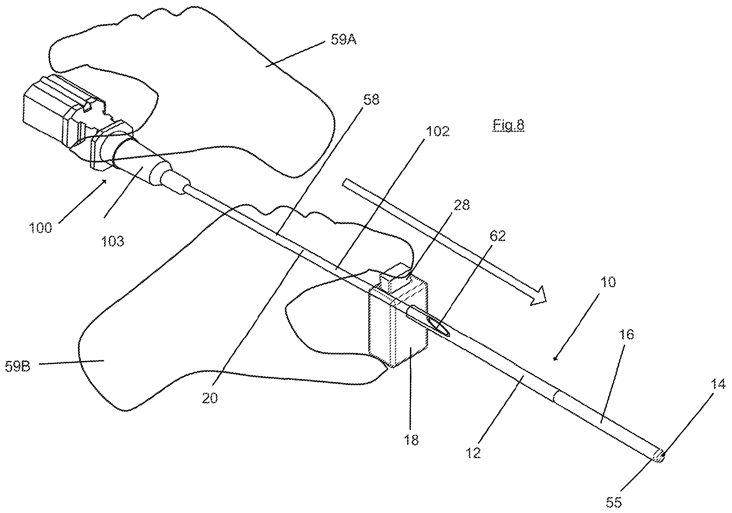

US2017/0112528A1 discloses another echogenic sheath (or cannula) assembly, see the sheath assembly 50 in fig. 8 and paragraphs 44-46 of the description. This concept is based on the idea of adding a connector 52 to the sheath assembly 50, wherein the connector is configured to be connected to the needle housing (hub) 40.

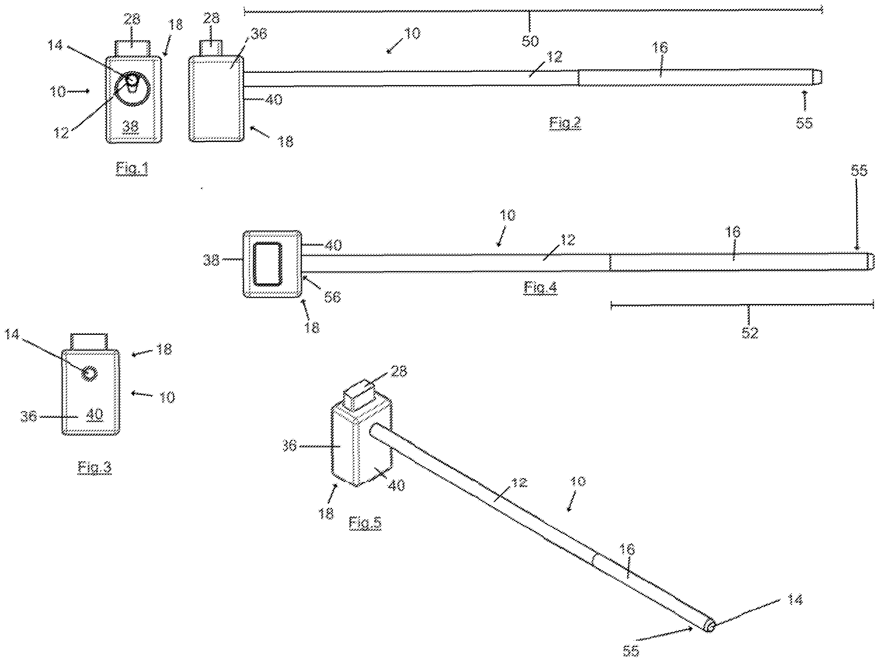

US 2012/059308 discloses another echogenic sheath (or cannula) assembly, see the sheath assembly 202 in fig. 2 and paragraphs 0039 of the description. This concept is also based on the idea of adding a connector 202 to the sheath assembly 100, wherein the connector is configured to be connected to the needle housing (hub) 200.

It was found in the present invention that all these sheaths have disadvantages. One relevant aspect that was recognized is that there are many different types of needles available on the market, in the order of hundreds or thousands of different types. As an example, for the biopsy system developed by the company BD/Bard, there are different versions called the Magnum and the Monopty. Each of these versions have a different housing (also called handle, luer lock, or hub) for the needle.

Many different suppliers of needles exist and each one generally manufactures its own versions. Therefore, a connector design which can be fixed to every needle handle is not possible from a practical point of view. Sheath assemblies could potentially be made separately for every single needle available on the market, and for every specified length and every found deviation in the specified length. However, this would result in having to produce hundreds if not thousands of different sheath types.

Furthermore, needle assemblies available on the market have different lengths. There is often a variation in needle length, e.g. a 11 cm needle from company X may be 11.1 cm, and a 11cm needle from company Y maybe 10.9 cm. A further aspect is that the tip of the sheath should be positioned close to the tip of the needle. The medical practitioner uses the echogenic tip of the sheath to position the needle tip at the desired location in the human body. When the echogenic tip of the sheath is too far from the tip of the needle, accurate positioning is not possible. Therefore, ideally it should be possible for a medical practitioner to position the tip of the sheath close to the tip of the needle. It was recognized that preferably the tip of the sheath exactly lines up with the proximal end of the bevel, so the doctor knows exactly where the needle tip is.

Different needles have different bevel types and varying bevel angles. The variation in bevel length and bevel angles makes alignment of the echogenic tip with the proximal bevel edge challenging.

One way of overcoming the problem of the variation in needle lengths would be to provide a sheath assembly which can be cut to length by the medical practitioner just prior to the actual use. However, it was recognized that the cutting operation itself may result in mechanical properties of the sheath assembly which are undesirable. For example, the tip of the sheath assembly may comprise splinters or fragments as a result of the cutting, and these splinters or fragments may come loose during use and stay behind in the body of the patient.

Also, as result of the cutting, the mechanical strength of the sheath may be compromised resulting in deformations when the sheath assembly is inserted into the body. For example, the sheath may wrinkle or buckle.

Also, a cutting operation may negatively affect the echogenicity of the sheath assembly.

It is also conceivable to provide a sheath assembly with breaking zones or breaking lines, allowing a user to break off a certain length of the sheath assembly in order to trim the sheath assembly to the required length. However, this provides the risk that in use, a part of the sheath assembly would break off inside the body of the patient which is under treatment. This is also undesirable.

Also, as result breaking zones or breaking lines, the mechanical strength of the sheath may be compromised resulting in deformations when the sheath assembly is inserted into the body. For example, the sheath may wrinkle or buckle.

These circumstances make it difficult to develop an echogenic sheath assembly capable of being used with a variety of needles, from different manufacturers.

US2008/009852 also discloses a sheath but provides no insight on how to fixate the sheath to the device.

A further problem or rather challenge associated with echogenic sheath assemblies, is that if the sheath would come loose from the needle assembly during use, this brings along with it a risk that the sheath would stay inside the body of the patient. The connector 52 disclosed in fig. 9 of US2017/0112528A1 may have this problem. It provides a solid connection when the needle is inserted into the body, but much less when the needle is pulled from the body. The sheath 50 may come loose and stay inside the body.

Leaving the sheath behind in the body may result in tissue irritation, an infection or internal bleedings and exposing a patient to an unacceptable health risk. Therefore, a more "general" connector which connects to various different types of housing (handle, luer lock, or hub) could be problematic, as unless used with the specific needle, there is increased chance of coming loose in use.

In addition if the sheath assembly moves during the procedure, the echogenic tip of the sheath may no longer be aligned with the proximal end of the needle bevel, causing the clinician to misposition the needle.

Therefore the connection between the sheath assembly and the needle assembly should be strong enough that once attached, the sheath does not move.
Clamps and connectors of various kinds are known from documents US 2017/135721A1,
US2013/261650A1, US5217438A1, US2001021824 A1 and CN109009359.

### OBJECT OF THE INVENTION

It is an object of the invention to provide an echogenic sheath assembly which is versatile and can be positioned over needles of different types, lengths and from different manufacturers. The echogenic sheath assembly should be safe in the sense that there is no risk or a negligible risk that the sheath comes loose from the needle assembly or catheter assembly. The sheath assembly should have good echogenic properties.

### SUMMARY OF THE INVENTION

The invention provides a sheath assembly configured to be positioned over a needle of a needle assembly or over a needle of a catheter assembly, the sheath assembly comprising an elongated tube and a connector, wherein the elongated tube has a defined bore and comprises an echogenic structure, wherein the echogenic structure is configured to be inserted into a body of a patient and to provide echogenicity inside the body, wherein the connector is configured to be fixated to an outside of the cannula wall of the needle, thereby fixating the sheath assembly.

This advantageously allows the sheath assembly to be connectable to the needle assembly independently of the design of a housing (handle, luer lock, or hub) of the needle assembly. The echogenic sheath assembly which connects directly to the needle shaft, solves several problems. The sheath assembly fits any manufacturer's needle regardless of housing or length variation. Moreover, the sheath assembly can be positioned at any point along the needle shaft to line up with the proximal end of the bevel.

In some embodiments, the connector comprises a clamp. This allows a strong gripping force on the outside of the cannula wall.

The clamp may comprise at least a first clamping member and a second clamping member which are movable relative to one another and are configured to clamp the outside of the cannula wall of the needle. In another embodiment, the connector may comprise a single clamping member which has an annular shape and is configured to circumferentially engage the outside of the cannula wall and wherein a circumferential length of the single clamping member can be increased or decreased in order to vary the clamping force on the outside of the cannula wall.

When seen in axial view, the clamp may be configured to engage the cannula wall in three locations, in particular at about 120 degrees from one another in the circumferential direction. However, it is also possible that the clamp engages the outside of the cannula wall in more locations, for instance 4 - 10 locations, or more.

In some embodiments, the connector comprises a biasing member configured for exerting a biasing force which pushes the first and second clamping member towards one another. It was found that the biasing force advantageously provides a very secure fixation.

In some embodiments, the connector comprises an operating member configured to be operated by the user, wherein the operating member is movable between a first position and a second position. In some embodiments, in the first position the connector is not fixated to the needle, allowing the sheath assembly to slide over the needle in order to be positioned or removed from the needle, and in the second position the connector is fixated to the needle. The operating member advantageously allows the user to easily fixate the sheath assembly to the needle.

In some embodiments, the connector comprises a first connector part comprising:
- the first and second clamping member, and
- external thread,
and a second, annular connector part comprising internal thread, wherein the second connector part is screwed onto the first connector part to press the first and second clamping member together against the outside of the cannula wall

In some embodiments, the operating member is movable in a direction orthogonal to a main axis of the needle. This direction results in a simple and yet at the same time reliable connector.

In some embodiments, the operating member is configured to move the first and second clamping member away from one another against the biasing force of the biasing member when the operating member is moved to the first position. Advantageously, the user actively loosens the sheath assembly while the biasing force automatically fixates the connector to the needle when the operating member is let go by the user.

In some embodiments, the connector is configured to fixate the sheath assembly to the needle via friction. It was found that friction provides a secure connection.

In some embodiments, the first and second clamping member each have a curved, concave clamping surface. Advantageously, the curved concave clamping surface matches with the shape of the needle. This results in a high friction force. Alternatively, the clamping surface may be straight.

In some embodiments, the first and second clamping member are provided with sharp protrusions or with a covering layer of anti-slip material. The sharp protrusions or anti-slip material advantageously improve the fixation.

In some embodiments, the second clamping member is a housing and wherein at least a part of the first clamping member is movably arranged within the housing. This may provide a simple and robust connector.

In some embodiments, the connector comprises a housing having a first hole in a first housing wall and a second hole in an opposite, second housing wall, wherein the first and second hole have an edge, wherein the edges define the first clamping member,
wherein the second clamping member is movably arranged inside the housing,
wherein the biasing member comprises a spring,
wherein the operating member moves the second clamping member from a clamping position to a disengaged position, wherein the spring biases the movable gripping member toward the clamping position.

This configuration provides a user-friendly embodiment which is very practical and reliable. Also the combination of the housing and the clamping member which is movably arranged within the housing allows a strong gripping force.

In some embodiments, the second clamping member comprises a clamping hole, wherein the first clamping member is movable between:
- the disengaged position in which the clamping hole lies entirely within a contour of the first hole and the second hole when seen in the axial direction of the sheath assembly, and
- the clamping position in which the clamping hole lies at least partially outside the contour of the first hole and the second hole when seen in the axial direction of the sheath assembly clamping hole.

The needle of the needle assembly or catheter assembly can be inserted through the clamping hole and is and supported in all lateral directions. Also a very strong gripping force is possible with this embodiment.

In some embodiments, the connector is not capable of being connected to a casing, housing, base, handle, hub or luer lock of a needle assembly.

In some embodiments, the biasing member extends around the housing and is connected to the operating member outside of the housing and to the housing itself. This embodiment allows a small housing in combination with a large and strong biasing member capable of exerting a gripping strong force to keep the sheath assembly in place.

In some embodiments, the sheath assembly is constructed and intended to be positioned over a needle or catheter assembly for biopsy, vascular access orperipheral nerve block applications. These were found to be the usages in which the sheath assembly provides the most benefits.

In various embodiments the one or more clamping member and the biasing member are integrated into a single resilient member. This results in a simple yet strong connector.

In one such embodiment, the single resilient member is a spring which defines an opening and is biased to a gripping position, wherein the spring comprises a first handle and a second handle configured to be pushed toward one another by a user with his or her fingers, thereby deforming the spring against a spring force to a more open form, wherein after the user lets go of the handles, the resilient member deforms back to a clamped position under the influence of the spring force. In another embodiment, the clamp may for instance be a ratchet clamp. In yet another embodiment, the clamp may be a slit clamp as explained further below.

In another embodiment, the clamp may be a hose clamp. Hose clamps are known to be very reliable and easy to operate.

In some embodiments, the echogenic structure comprises a material with a different acoustic impedance than the tissue: fat, vein, artery, organ or muscle or any other body part into which the needle is inserted. The difference in acoustic impedance between the tissues fat, vein, organ or muscle and the echogenic structure causes the ultrasound signal to be reflected back to the ultrasound transducer, and an image of the device to appear on the ultrasound screen. The material of the echogenic structure may be harder than any tissue of the human body, e.g. glass which has an acoustic impedance of about 145 × 10⁷ kg/m2.s . Alternatively, the material may be air or another gas. Air has an acoustic impedance 0.0004 × 10⁶ kg/m2.s. of Both a hard material and air or another gas have an acoustic impedance which is very different from the tissue of the human body.

In some embodiments, the echogenic structure is incorporated in a coating which is applied on the elongated tube.

In an embodiment, the coating comprises small particles, said small particles being manufactured from a material different than, the coating layer itself.

In some embodiments, the particles are gas, solid, gel or liquid particles.

In some embodiments, the particles are solid, gel or liquid particles, in particular glass, sand or crystal particles.

In some embodiments, the echogenic structure is not applied over the full length of the elongated tube, but only over a part of the length thereof. It was found that applying the echogenic structure over the distal end of the elongated tube improves the medical practitioner's ability to locate the tip.

In some embodiments, the coated echogenic structure has a length which at most extends over 50 percent of the length of the elongated tube, in particular over at most 25 percent, more in particular over at most 10 percent of the length of the elongate tubule. This ensures that the medical practitioner can clearly identify the needle tip.

In some embodiments, the echogenic structure is located at a distal end of the sheath and not at a proximal end of the sheath, in particular at the end of the elongated tube which is opposite to the end where the connector is located. It was advantageously found that the echogenic structure only needs to be applied at the tip of the elongated tube in order to allow the medical practitioner to accurately position the needle inside the body of the patient.

In some embodiments, the coated echogenic structure has a length of less than 5 cm. It was found that this length is sufficient for a medical practitioner to see the tip of the sheath assembly on the screen and to accurately position the needle on the basis of this information.

In some embodiments, the particles are solid microspheres. Solid microspheres advantageously reflect the sound waves in all directions. In this way, the sheath assembly is visible for the medical practitioner from all sides.

In some embodiments, the echogenic structure is manufactured from metal.

In some embodiments, the elongated tube comprises a tube wall which comprises a first, inner layer and a second, outer layer, and wherein the echogenic structure is:
- located between the inner and outer layer, or
- provided on the outer side of the inner layer, or
- provided on the inner side of the outer layer.

In some embodiments, the elongated tube is made from a polymer tubing, in particular Polyurethane, PolyEther Ketone, Polyether ether ketone, PEVAc, POM, PTSE, PEBAX, Nylon, PET, polyimide, polyamide, polyester, polycarbonate, PVC, polyethylene, polypropylene, FEP, silicone.

In some embodiments, the echogenic structure comprises gas bubbles inside the tube wall of the elongated tube or an echogenic structure adhesively bonded to the tube wall of the elongated tube.

In some embodiments, the echogenic structure comprises small particles, inside the tube wall of the elongated tube, the small particles being manufactured from a material different than the elongated tube wall itself.

In some embodiments, the particles are gas, solid, gel or liquid particles, preferably glass particles.

In some embodiments, the proximal end of the elongated tube at the connector tapers outwardly and distal end of the elongated tubed does not taper outwardly.

In some embodiments, the echogenic structure comprises a coating and solid microspheres embedded in the coating layer, wherein the echogenic structure is only provided at the distal end of the elongated tube, and wherein the connector comprises a clamp comprising a first clamping member and a second clamping member and a biasing member configured for exerting a biasing force which pushes the first and second clamping member towards one another.

The present invention further relates to a set of a sheath assembly according to the invention and a catheter assembly or needle assembly, wherein a shape of the connector corresponds with a radius of the outside of the cannula wall of the catheter assembly or needle assembly, wherein in particular a radius of the concave clamping surface of the first and second clamping member corresponds to a radius of the outside of the cannula wall .

In some embodiments of the set, the concave clamping surface of the first and second clamping member have a radius which is 90-110 percent of the radius of the outside of the cannula wall.

In some embodiments of the set, the connector is not capable of being connected to a casing, housing, base, handle, hub or luer lock of the needle assembly or catheter assembly.

The needle of the set may comprise notches in the outside of the cannula wall configured for providing grip for the connector of the sheath assembly. This may improve the fixation of the connector onto the needle.

The present invention further relates to a method for making a sheath assembly according to the invention, the method comprising:
- providing an elongated tube having an echogenic structure,
- connecting said elongated tube to a connector configured to be fixated to an outside of a cannula wall of a needle, thereby fixating the sheath assembly to the needle.

These and other aspects of the invention can be better understood by reference to the following detailed description and the accompanying drawings. In the accompanying drawings like reference symbols designate like parts.

### SHORT DESCRIPTION OF THE FIGURES

Figure 1 shows a front side of an embodiment of the sheath assembly according to the invention.
Figure 2 shows a side view of an embodiment of the sheath assembly according to the invention.
Figure 3 shows a rear view of an embodiment of the sheath assembly according to the invention.
Figure 4 shows a top view of an embodiment of the sheath assembly according to the invention.
Figure 5 shows an isometric view of an embodiment of the sheath assembly according to the invention.
Figure 6 shows a sectional front view of the sheath assembly according to the invention.
Figure 7 shows a sectional side view of the sheath assembly according to the invention.
Figure 8 shows an isometric view of the embodiment of figures 1 - 5 in operation.
Figure 9 shows a top view of a set comprising the sheath assembly according to the invention and a needle assembly.
Figure 10 shows a side view of the set of figure 7.
Figure 11 shows an isometric view of the set of figures 7 and 8.
Figures 12 - 17 show a further embodiment of the invention.
Figures 18 - 22 show a further embodiment of the invention which is a variant of the embodiment of figures 12 - 17.
Figures 23 - 31 show a further embodiment of the invention.
Figure 32 shows yet another embodiment of the invention.
Figures 33 - 37 show another embodiment of the invention, wherein figure 33 comprises figure 33A and figure 33B.
Figures 38-41 show again another embodiment of the invention.
Figures 42-46 show yet another embodiment of the invention.
Figures 47-51 show a further embodiment of the invention.
Figures 52-57 show again another embodiment of the invention.
Figures 58-60 shows further embodiments of the invention in which the echogenic structure comprises metal, particles or bubbles.
Figure 61 shows a further embodiment of the invention.

### Detailed description of the figures

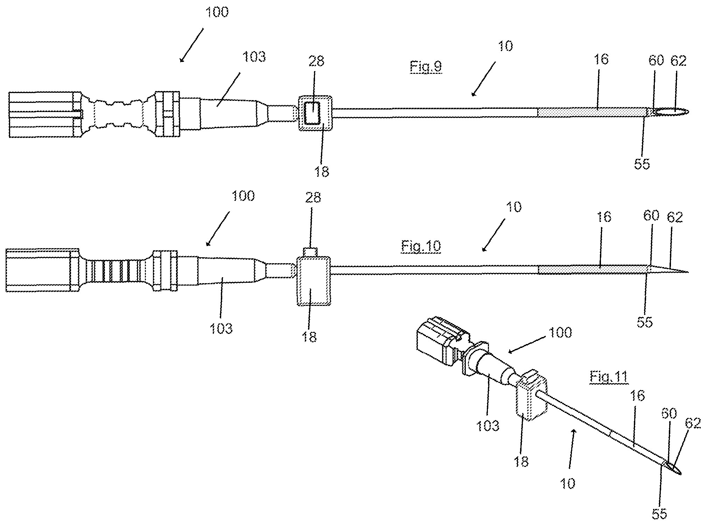

Turning to figures 1-11, a sheath assembly 10 is shown which is configured to be positioned over a needle 102 of a needle assembly 100 or over a needle of a catheter assembly. The sheath assembly 10 comprises an elongated tube 12 (also called sheath 12) which defines a bore 14 and an echogenic structure 16.

The sheath assembly further comprises a connector 18 configured to fixate the sheath assembly to the outside of the cannula wall 20 of the needle 102. The connector is positioned at a proximal end of the sheath assembly.

The connector 18 is relatively small in order not to hinder the user or the patient. The outer dimensions may be smaller than 20 mm (length) by 20 mm (width) by 30 mm (height). In this way almost the full needle length or catheter length can be used.

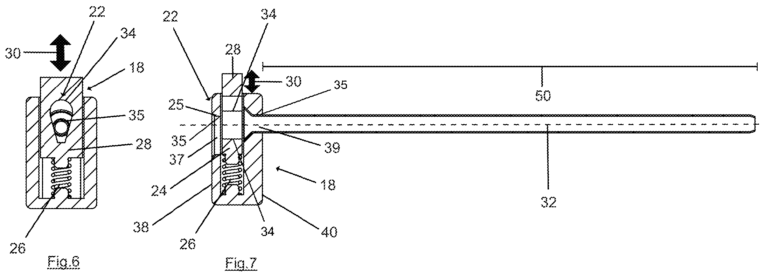

With particular reference to figures 6 and 7, the connector 18 comprises a clamp 22 comprising at least a first clamping member 24 and a second clamping member 25 and a biasing member 26 configured for exerting a biasing force which pushes the first and second clamping member towards one another.

The connector 18 further comprises an operating member 28 configured to be operated by the user, wherein the operating member 28 is movable in a direction 30 between a first position and a second position, wherein in the first position the connector 18 is not fixated to the needle 102, allowing the sheath assembly to slide over the needle in order to be positioned or removed from the needle, and wherein in the second position the connector is fixated to the needle.

The operating member 18 is movable in a direction 30 orthogonal to a main axis 32 of the elongated tube 12.

The operating member 18 is configured to move the first and second clamping member 24, 25 away from one another against the biasing force of the biasing member 26 when the operating member is moved to the first position.

The connector 18 is configured to fixate the sheath assembly to the needle via friction.

At least one of the first and second clamping member 24, 25 may have a curved, concave clamping surface 34, 35.

The first and second clamping member 24, 25 may be provided with sharp protrusions or with a covering layer of anti-slip material.

The connector comprises a housing 36 having a first hole 37 in a first housing wall 38 and a second hole 39 in an opposite, second housing wall 40. The elongated tube 12 is connected to the second housing wall 40.

The first hole 37 and second hole 39 each have an edge, wherein the edges define the second clamping member 25, wherein the first clamping member 24 is movably arranged inside the housing 36, wherein the biasing member 26 comprises a spring, wherein the operating member moves the first clamping member from a clamping position to a disengaged position, wherein the spring biases the movable gripping member toward the clamping position.

The first clamping member 24 comprises a clamping hole 42, wherein the first clamping member is movable between:
- the disengaged position in which the clamping hole 42 is aligned with the first hole 37 and the second hole 39, and
- the clamping position in which the clamping hole 42 is not aligned with the first hole 37 and the second hole 39.

When seen in side view, see fig. 7, the second clamping member 25 comprises a first clamping surface 35 and a second clamping surface 35 located at a distance from one another and associated with respectively the first hole 37 and the second hole 39. The first and second clamping surface 35 engage the outside of the cannula wall from one side. The clamping surface 34 of the first clamping member 24 engages the outside of the cannula wall from the opposite side. In side view, the clamping surface 34 of the first clamping member 24 is located between the first and the second clamping surface 35. In this way the clamp provides a three point clamping arrangement. Obviously, the clamp may engage the outside of the cannula wall in more than three locations, when seen in side view.

The connector 18 is not capable of being connected to a casing 103, housing, base, handle, hub or luer lock of a needle assembly, only to the needle 102 itself. The connector may abut the casing, housing, base, handle, hub or luer lock of a needle assembly, though.

The sheath assembly 10 may in particular be constructed and intended to be positioned over a needle of a needle assembly for vascular access, or over a needle of a catheter assembly for a biopsy or for a peripheral nerve block.

The echogenic structure 16 comprises a coating which is applied on the elongated tube 12.

The echogenic structure 16 comprises the coating and small particles, said small particles being manufactured from a material with an different acoustic impedance than the coating layer itself, the tissue, fat, vein, artery, organ or muscle or any other body part into which the needle is inserted, wherein the ultrasound signal is reflected back to the ultrasound transducer, and an image of the device appears on the ultrasound screen .

The particles are gas, solid, gel or liquid particles. The particles may in particular be glass, sand or crystal particles. The particles may be solid microspheres.

The echogenic structure 16 is not applied over the full length 50 of the elongated tube 12, but only over a part 52 of the length thereof. The length 50 of the elongated tube may be 45-55 mm, in particular about 49mm as shown in fig. 7. However, for different applications, different lengths may be used.

The echogenic structure has a length 52 which extends over at most 50 percent of the length of the elongated tube, in particular over at most 25 percent, more in particular over at most 10 percent of the length of the elongate tubule. The echogenic structure may have a length 52 of less than 3.5 cm.

The echogenic structure is located at a distal end 55 of the sheath and not at a proximal end 56 of the sheath, in particular at the end of the elongated tube 12 which is opposite to the end where the connector 18 is located.

In addition or in an alternative variant the echogenic structure or a part thereof is manufactured from metal.

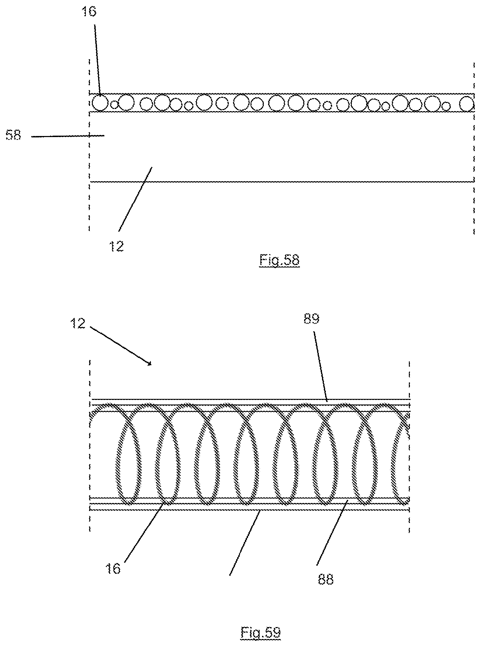

The elongated tube 12 may comprise a tube wall 58 which comprises a first, inner layer 88 and a second, outer layer 89, and wherein the echogenic structure is:
- located between the inner and outer layer, or
- provided on the outer side of the inner layer, or
- provided on the inner side of the outer layer.
This is further discussed in relation to fig. 59.

The elongated tube 12 may be made from a polymer tubing, in particular Polyurethane, PolyEther Ketone, Polyether ether Ketone, PEVAc, POM, PTSE, PEBAX, Nylon, PET, polyimide, polyamide, polyester, polycarbonate, PVC, polyethylene, polypropylene, FEP, silicone.

Additionally or alternatively, the echogenic structure may comprise gas bubbles inside the tube wall of the elongated tube or a echogenic structure adhesively bonded to the tube wall of the elongated tube.

The distal end 55 of the tube 12 does not taper outwardly.

The echogenic structure may comprise a coating and solid microspheres embedded in the coating layer, wherein the echogenic structure is only provided at one end, the distal end, of the elongated tube and has a length 52 of less than 20 percent of the length 50 of the elongated tube 12, the length 52 being less than 5cm. The connector 18 comprises a clamp comprising a first clamping member 24 and a second clamping member 25 and a biasing member 26 configured for exerting a biasing force which pushes the first and second clamping member towards one another.

The present invention also relates to a set 200 of a sheath assembly 10 according to any of the preceding claims 2 - 31 and a catheter assembly or needle assembly 100, wherein a shape of the connector 18 corresponds with a radius r1 of the outside of the cannula wall 58 of the catheter assembly or needle assembly. In particular a radius r2 of the concave clamping surfaces 34, 35 of the first and second clamping member corresponds to the radius r1 of the outside of the cannula wall.

The concave clamping surfaces 34,35 of the first and second clamping member 24, 25 may have a radius which is 90-110 percent of the radius of the outside of the cannula wall.

The connector 18 may not be capable of being connected to a casing, housing, base, handle, hub or luer lock of the needle assembly or catheter assembly which is part of the same set.

A method for making a sheath assembly according to any the invention may comprise:
- providing an elongated tube having an echogenic structure,
- connecting said elongated tube to a connector configured to be fixated to an outside of a cannula wall of a needle, thereby fixating the sheath assembly to the needle.

### Operation

Turning in particular to figures 8-11, in operation, a medical practitioner may have a set as disclosed above comprising a needle assembly 100 or catheter assembly on the one hand and a sheath assembly 10 on the other hand.

The sheath assembly may come in a separate package and may not be specifically designed for the exact length of the needle of the needle assembly or catheter assembly. Instead, the sheath assembly 10 can be used for a range of needle assemblies 100 or catheter assemblies.

The medical practitioner holds the needle assembly 100 or catheter assembly with one hand 59A, takes the sheath assembly 10 with his other hand 59B, and pushes the operating member 28 downward and slides the elongated tube 12 over the needle 102 until the tip 55 (also called the distal end 55) is positioned just at the edge 60 of the bevel 62 of the needle 102. The medical practitioner can check this visually. In this position, the user releases the operating member 28 and the sheath assembly is fixated to the needle 102.

The completed assembly is now ready for use. The medical practitioner can insert the needle 102 with the sheath into the patient. The ultrasound signal is reflected back to the ultrasound transducer, and an image of the device appears on the screen of an ultrasound imaging device. The echogenic structure allows the user to see where the needle tip is inside the body and to controllably move the needle tip inside the body.

When the medical practitioner is ready with the treatment, he or she can retrieve the completed assembly from the body by pulling back the needle, The sheath assembly 10 will automatically also be pulled back as a result of the connector 18 which provides the fixation.

Because the connector can be positioned anywhere along the needle 102, there is no need for cutting the sheath 12 to length.

### Further embodiments

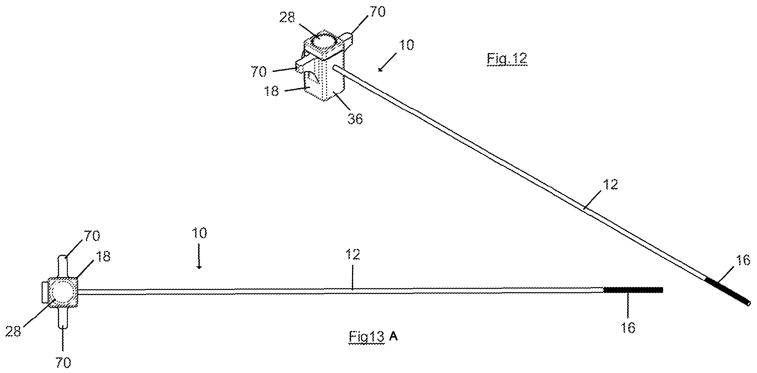

Turning to figures 12-17, in another embodiment, the connector 18 comprises one or more finger grips 70 which protrude from the housing. The second hole 39 tapers outwardly.

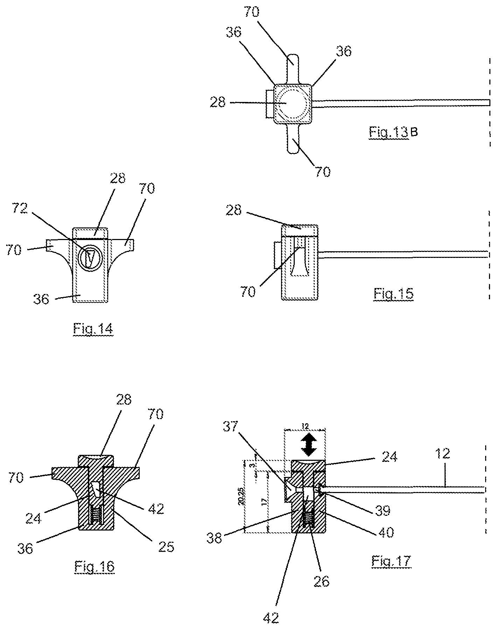

The clamping hole 42 has a shape of a triangle or a triangle with a chamfered corner. The first and second holes 37, 39 may have a similar shape but mirrored. As can be seen in figure 14 these two shapes together define a triangular through passage 72 which in use defines three contact points with the needle. In other words, the clamp is configured to engage the cannula wall in three locations. These three locations may in particular be located at about 120 degrees from one another in the circumferential direction.

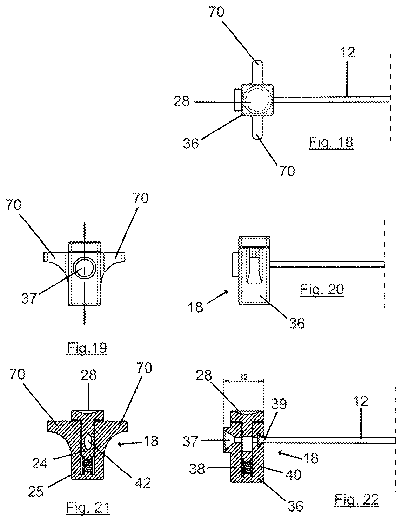

Turning to the embodiment of figures 18-22, this embodiment is essentially the same as the embodiment of figures 12-17, except that the clamping hole 42 is oval. The first and second holes 37, 39 are circular.

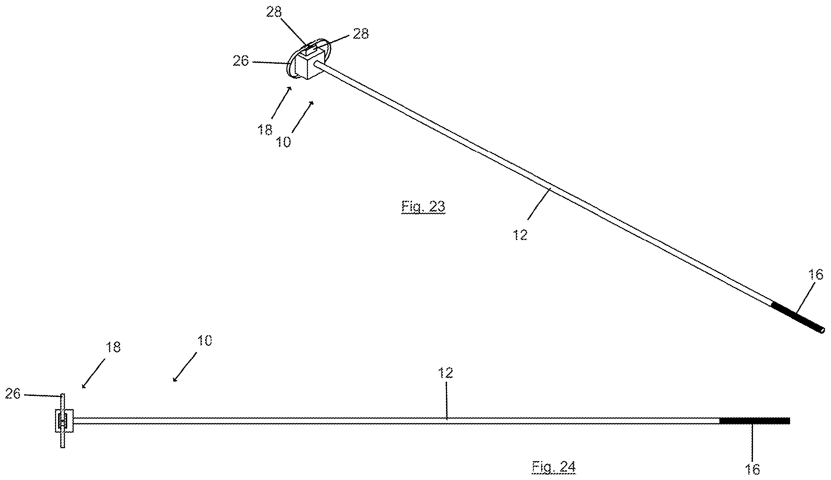

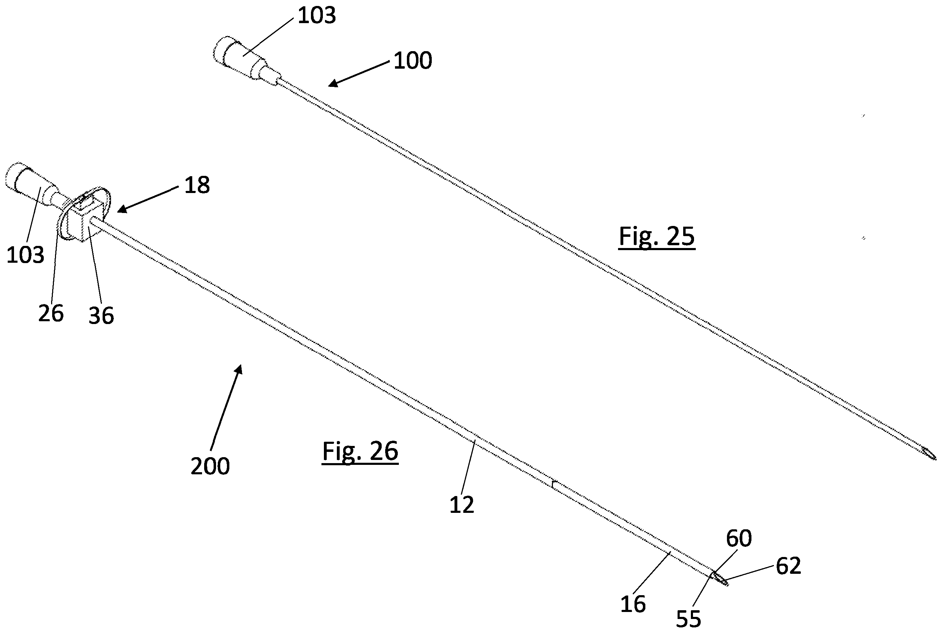

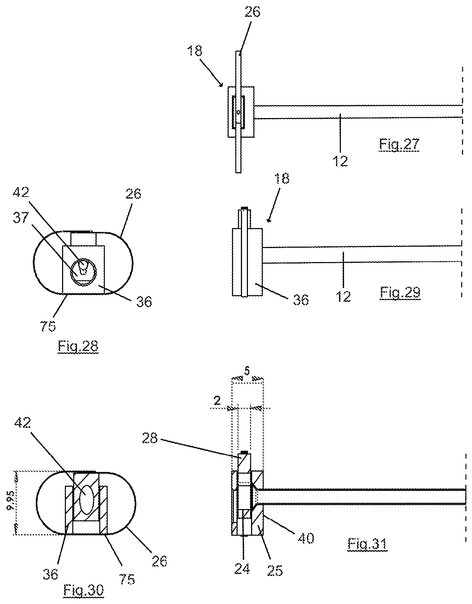

Turning to the embodiment of figures 23-31, in this embodiment the biasing member 26 is located outside the housing 36. The biasing member 26 extends around the housing 36 and is connected to the operating member 28 outside of the housing. The biasing member is also connected to the housing 36 itself, in particular to a bottom side 75 of the housing. The biasing member may have an oval shape. This makes it possible to apply a large and strong biasing member.

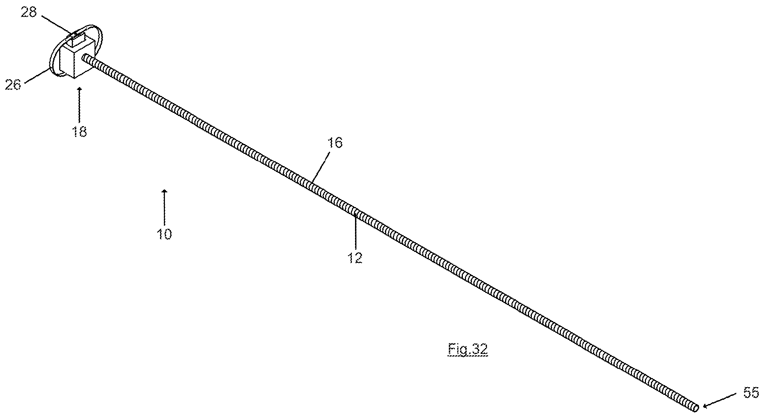

Turning to figure 32, in another embodiment, the echogenic structure 16 may be a metal structure extending around the tube 12. In particular the echogenic structure may be a metal coil which spirals around the tube 12. The tube 12 may have a first inner layer and a second outer layer and the echogenic structure may be located between the first and second layer. The metal coil may only extend over a portion of the length of the tube 12, or may extend over the entire length of the tube 12.

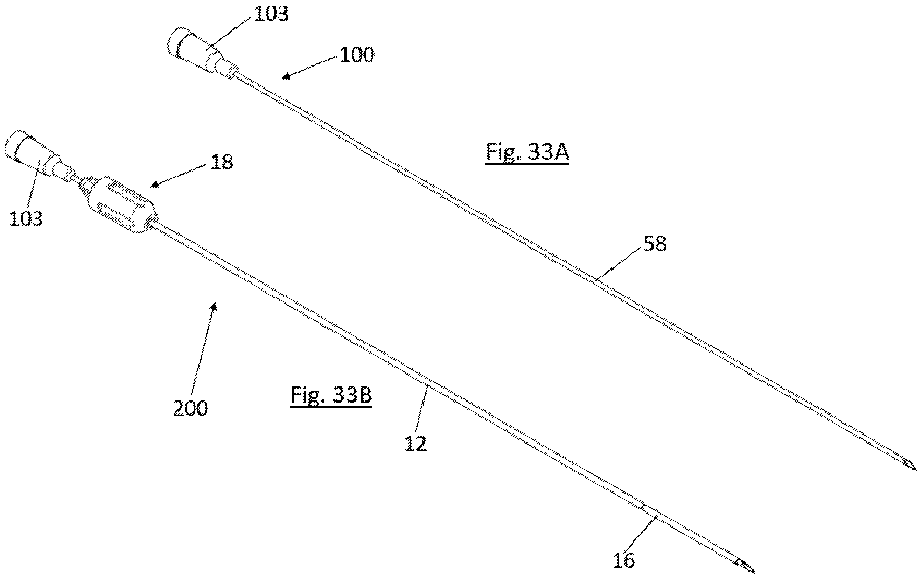

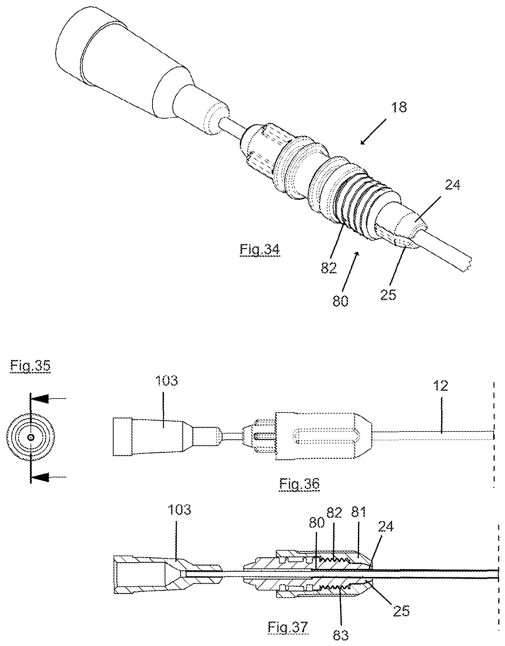

Turning to figures 33 - 37, in another embodiment, the connector 18 comprises first connector part 80 comprising:
- the first and second clamping member 24, 25, and
- external thread 82,
and a second, annular connector part 81 comprising internal thread 83, wherein the second connector part is screwed onto the first connector part to press the first and second clamping member 24, 25 together against the outside of the cannula wall. The tube 12 is connected to the first connector part 80. This allows a very strong fixation of the sheath assembly onto the needle assembly or catheter assembly.

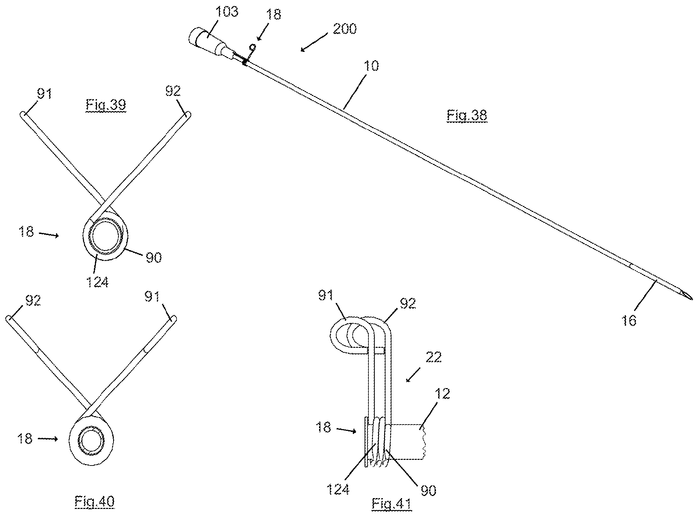

Turning to figures 38 - 41, in another embodiment, the connector comprises a clamp having a single clamping member 124 which has an annular shape and is configured to circumferentially engage the outside of the cannula wall. A circumferential length of the single clamping member can be increased or decreased in order to vary the clamping force on the outside of the cannula wall. In this case the single clamping member 124 is a coiled spring 90.

The spring 90 defines an opening and is biased to a gripping position. The spring comprises a first handle 91 and a second handle 92 configured to be pushed toward one another by a user with his or her fingers, thereby deforming the spring against a spring force to a more open form having a greater circumferential length, wherein after the user lets go of the handles 91, 92, the resilient member deforms back to a clamped position under the influence of the spring force. The handles are connected to opposite ends of the coiled spring.

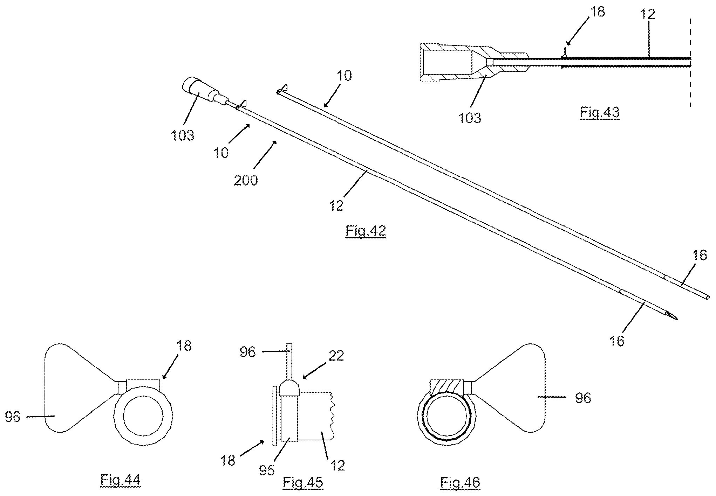

Turning to figures 42-46, in another embodiment, the clamp 22 is a hose clamp and comprises a strip 95 defining a circumference around an opening and an adjustment device 96, in particular a rotary device. The adjustment device 96 engages a first portion of the strip and a second portion of the strip, wherein the adjustment device is configured to adjust a circumferential length of the strip, thereby controlling the fixation of the clamp 22 on the outside of the cannula wall .

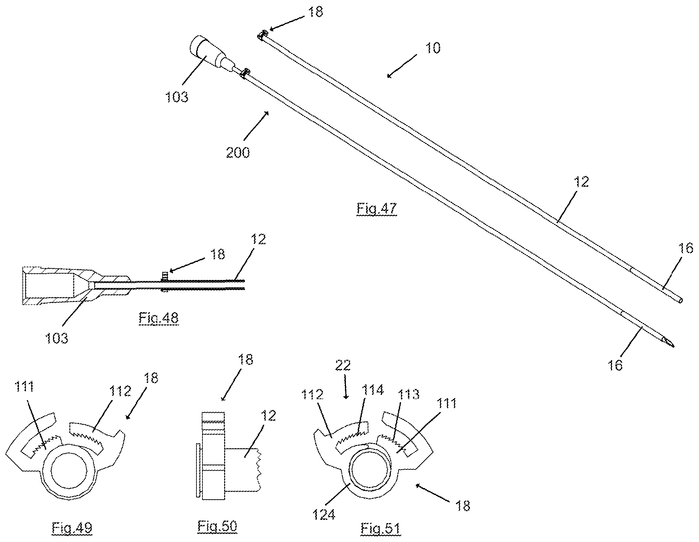

Turning to figures 47-51, in another embodiment, the connector 18 comprises a ratchet clamp 22. The ratchet clamp 22 is self locking and comprises a single clamping member 124 which has an annular shape and is configured to circumferentially engage the outside of the cannula wall. The resilient body 124 defines an opening and extends at least partially around said opening. The ratchet clamp comprises a first ratchet member 111 and a second ratchet member 112 configured to engage one another and comprising respectively a first ratchet 113 and a second ratchet 114. The user can simply press the first and second ratchet members together, thereby fixating the connector 18 onto the outside of the cannula wall.

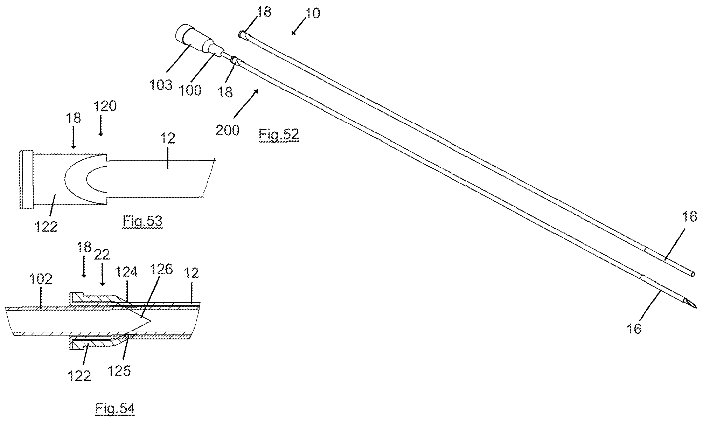

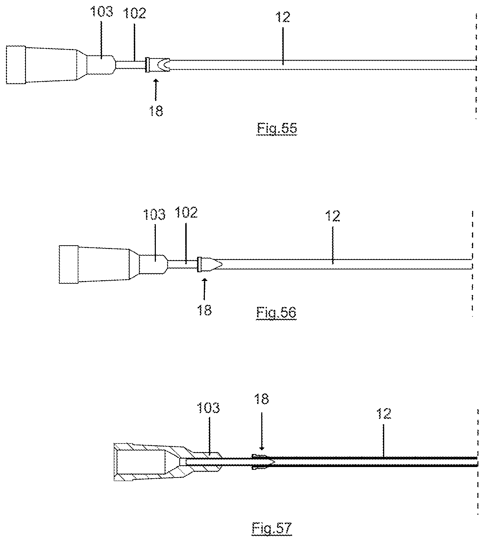

Turning to figures 52-57, in this embodiment, the clamp 22 comprises a body 120 comprising:
- a tubular base 122 which defines a base bore, and
- an upper slit part 124 and a lower slit part 125 which define a slit 126 between them.

The upper and lower slit part 124, 125 extend from the tubular base towards the distal end 55 of the sheath assembly 10. When viewed in an axial direction of the sheath assembly 10 the first and second slit part 124, 125 are located within the bore of the elongated tube 12. The first and second slit part 124, 125 are resilient and allow the needle 102 to slide through the slit 126 in one direction but resist a sliding movement of the needle in an opposite direction.

Turning to figure 58, in a further embodiment, the echogenic structure 16 comprises particles or gas bubbles 90 inside the sheath 12. In case of gas bubbles, the gas may be air. In case of particles, the particles are manufactured from a material different than the tube wall itself, for instance glass particles. The bubbles or particles may have a same size or have different sizes. The bubbles or particles may have various shapes such as spheres, cubes, cylinders, polyhedrons or irregular shapes. The bubbles or particles may be arranged in an orderly manner.

Turning to figure 59, in another embodiment, the echogenic structure comprises a metal material located between an inner layer 88 and an outer layer 89. The echogenic structure can also be located on the outer side of the inner layer, or on the inner side of the outer layer. The metal echogenic structure can be in the shape of a coil or a braid.

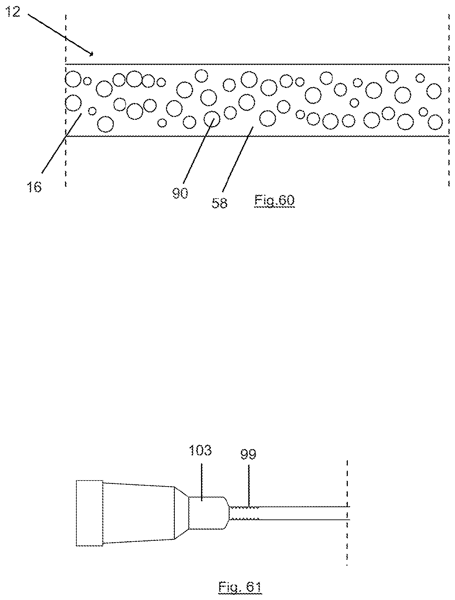

Turning to figure 60, in another embodiment the particles or bubbles 90 may have varying sizes and may be randomly dispersed in the cannula wall. Obviously, the particles and/or bubbles may also have uniform sizes (and shape).

Turning to figure 61, in another embodiment, the needle of the needle assembly has ridges or grooves (99) or more generally a roughened surface for instance in the form of a pattern. This may further improve the gripping connection. The notches or grooves may extend circumferentially. A plurality of circumferential notches or grooves may be provided. The ridges, grooves or roughened surface extends over a limited length in the axial direction.

It will be clear to the skilled person that different embodiments of the connectors can be combined with different embodiments of the echogenic structure.

The terms "a" or "an", as used herein, are defined as one or more than one. The term plurality, as used herein, is defined as two or more than two. The term another, as used herein, is defined as at least a second or more. The terms including and/or having, as used herein, are defined as comprising i.e., open language, not excluding other elements or steps.

The mere fact that certain measures are recited in mutually different dependent claims does not indicate that a combination of these measures cannot be used to advantage.

White lines between text paragraphs in the text above indicate that the technical features presented in the paragraph may be considered independent from technical features discussed in a preceding paragraph or in a subsequent paragraph.

## Claims

1. Sheath assembly (10) configured to be positioned over a needle (102) of a needle assembly (100) or over a needle of a catheter assembly, the sheath assembly comprising an elongated tube (12) and a connector (18),
wherein the elongated tube has a defined bore (14) and comprises an echogenic structure (16), wherein the echogenic structure is configured to be inserted into a body of a patient and to provide echogenicity inside the body,
**characterized in that**
the connector (18) is configured to be fixated to an outside of the cannula wall of the needle, thereby fixating the sheath assembly.

2. Sheath assembly according to claim 1, wherein the connector comprises a clamp (22) configured to exert a clamping force on the outside of the cannula wall of the needle, wherein the clamp comprises at least a first clamping member (24) and a second clamping member (25) which are movable relative to one another and are configured to clamp the outside of the cannula wall of the needle,
or a single clamping member which has an annular shape and is configured to circumferentially engage the outside of the cannula wall and wherein a circumferential length of the single clamping member can be increased or decreased in order to vary the clamping force on the outside of the cannula wall, wherein the connector comprises a biasing member (26) configured for exerting a biasing force which pushes the clamping members towards one another.

3. Sheath assembly according to any of claims 1-2, wherein the connector comprises at least one operating member (28) configured to be operated by the user, wherein the operating member is movable between a first position and a second position, wherein in the first position of the operating member the connector is not fixated to the needle, allowing the sheath assembly to slide over the needle in order to be positioned or removed from the needle, and wherein in the second position the connector is fixated to the needle.

4. Sheath assembly according to claim 3, wherein the operating member (28) is configured to move the first and second clamping member away from one another against the biasing force of the biasing member when the operating member is moved to the first position.

5. Sheath assembly according to any of the preceding claims, wherein the connector is not capable of being connected to a casing, housing, base, handle, hub or luer lock of a needle assembly.

6. Sheath assembly according to any of the preceding claims, constructed and intended to be positioned over a needle of a needle assembly for vascular access, a biopsy or for a peripheral nerve block.

7. Sheath assembly according to any of the preceding claims, wherein the echogenic structure comprises a coating which is applied on the elongated tube.

8. Sheath assembly according to the preceding claims 1-6, wherein the echogenic structure comprises a coating, wherein the coating comprises small particles having a different acoustic impedance than the coating layer itself and tissues of the human body.

9. Sheath assembly according to any of the preceding claims, wherein a material of the echogenic structure has a different acoustic impedance than human tissue.

10. Sheath assembly according to any of the preceding claims, wherein the echogenic structure comprises a material that has a surface which provides different angles of reflectance of incoming sound waves, resulting in the sound waves being scattered in every direction.

11. Sheath assembly according to any of the preceding claims, wherein the echogenic structure is not applied over the full length of the elongated tube, but only over a part of the length thereof, wherein the echogenic structure has a length (L) which extends over at most 50 percent of the length of the elongated tube.

12. Sheath assembly according to the preceding claim, wherein the echogenic structure is located at a distal end (55) of the sheath and not at a proximal end (56) of the sheath, wherein the echogenic structure is located at the end of the elongated tube which is opposite to the end where the connector is located.

13. Sheath assembly according to any of the preceding claims 1-6 or claims 9-12, wherein the echogenic structure is at least partially manufactured from metal, wherein the metal echogenic structure is not a coating, and wherein the metal echogenic structure comprises a coil or a braid.

14. Sheath assembly according to any of the preceding claims 1-6 or claims 9-12, wherein the echogenic structure comprises gas bubbles inside the tube wall of the elongated tube or an echogenic structure adhesively bonded to the tube wall of the elongated tube or wherein the echogenic structure comprises particles, in particular liquid, solid or gel particles, or gas filled bubbles inside the wall of the elongated tube.

15. Combination (200) of a sheath assembly (10) according to any of the preceding claims 2 - 14 and a catheter assembly or needle assembly (100), wherein a shape of the clamping member corresponds with a radius of the outside of the cannula wall of the catheter assembly or needle assembly, the clamping member being concave according to a radius of curvature.

16. Combination of claim 15, wherein the needle comprises ridges on the outside or grooves in the outside of the cannula wall configured for providing grip for the connector of the sheath assembly or a cannula wall having a section with a roughened surface.

17. Method for making a sheath assembly according to any of the preceding claims 1-14, the method comprising:
- providing an elongated tube having an echogenic structure,
- connecting said elongated tube to a connector configured to be fixated to an outside of a cannula wall of a needle, thereby fixating the sheath assembly to the needle.

## Patentansprüche

1. Hüllenanordnung (10), die dazu ausgelegt ist, über eine Nadel (102) einer Nadelanordnung (100) oder über eine Nadel einer Katheteranordnung positioniert zu werden, wobei die Hüllenanordnung eine längliche Röhre (12) und einen Verbinder (18) umfasst,
wobei die längliche Röhre eine definierte Bohrung (14) hat und eine echogene Struktur (16) umfasst, wobei die echogene Struktur dazu ausgelegt ist, in einen Körper eines Patienten eingesetzt zu werden und in dem Körper Echogenizität bereitzustellen,
**dadurch gekennzeichnet, dass**
der Verbinder (18) dazu ausgelegt ist, an einer Außenseite der Kanülenwand der Nadel fixiert zu werden und dadurch die Hüllenanordnung zu fixieren.

2. Hüllenanordnung nach Anspruch 1, wobei der Verbinder eine Klemme (22) umfasst, die dazu ausgelegt ist, eine Klemmkraft auf die Außenseite der Kanülenwand der Nadel auszuüben, wobei die Klemme mindestens ein erstes Klemmelement (24) und ein zweites Klemmelement (25) umfasst, die bezogen aufeinander beweglich und dazu ausgelegt sind, die Außenseite der Kanülenwand der Nadel zu klemmen,
oder ein einzelnes Klemmelement, das eine ringförmige Form hat und dazu ausgelegt ist, die Außenseite der Kanülenwand umlaufend in Eingriff zu nehmen und wobei eine Umfangslänge des einzelnen Klemmelements erhöht oder reduziert werden kann, um die Klemmkraft auf der Außenseite der Kanülenwand zu variieren, wobei der Verbinder ein Vorspannelement (26) umfasst, das zum Ausüben einer Vorspannkraft ausgelegt ist, die die Klemmelemente aufeinander zu drückt.

3. Hüllenanordnung nach einem der Ansprüche 1-2, wobei der Verbinder mindestens ein Bedienelement (28) umfasst, das dazu ausgelegt ist, von dem Benutzer bedient zu werden, wobei das Bedienelement zwischen einer ersten Position und einer zweiten Position beweglich ist, wobei in der ersten Position des Bedienelements der Verbinder nicht an der Nadel fixiert ist, wodurch die Hüllenanordnung über die Nadel gleiten kann, um über der Nadel positioniert oder davon entfernt zu werden, und wobei in der zweiten Position der Verbinder an der Nadel fixiert ist.

4. Hüllenanordnung nach Anspruch 3, wobei das Bedienelement (28) dazu ausgelegt ist, das erste und das zweite Klemmelement gegen die Vorspannkraft des Vorspannelements voneinander weg zu bewegen, wenn das Bedienelement in die erste Position bewegt wird.

5. Hüllenanordnung nach einem der vorhergehenden Ansprüche, wobei der Verbinder nicht mit einer Ummantelung, einem Gehäuse, einer Basis, einem Griff, einem Ansatz oder einem Luer-Anschluss einer Nadelanordnung verbunden werden kann.

6. Hüllenanordnung nach einem der vorhergehenden Ansprüche, die dazu ausgebildet und gedacht ist, über eine Nadel einer Nadelanordnung für Gefäßzugang, eine Biopsie oder für eine periphere Nervenblockade positioniert zu werden.

7. Hüllenanordnung nach einem der vorhergehenden Ansprüche, wobei die echogene Struktur eine Beschichtung umfasst, die auf die längliche Röhre aufgebracht ist.

8. Hüllenanordnung nach einem der vorhergehenden Ansprüche 1-6, wobei die echogene Struktur eine Beschichtung umfasst, wobei die Beschichtung kleine Partikel mit einer anderen akustischen Impedanz als die Beschichtung selbst und Gewebe des menschlichen Körpers umfasst.

9. Hüllenanordnung nach einem der vorhergehenden Ansprüche, wobei ein Material der echogenen Struktur eine andere akustische Impedanz als menschliches Gewebe hat.

10. Hüllenanordnung nach einem der vorhergehenden Ansprüche, wobei die echogene Struktur ein Material umfasst, das eine Oberfläche hat, die andere Reflexionswinkel eingehender Schallwellen bereitstellt, was darin resultiert, dass die Schallwellen in alle Richtungen gestreut werden.

11. Hüllenanordnung nach einem der vorhergehenden Ansprüche, wobei die echogene Struktur nicht über der gesamten Länge der länglichen Röhre aufgebracht ist, sondern nur über einem Teil der Länge davon, wobei die echogene Struktur eine Länge (L) hat, die sich über maximal 50 Prozent der Länge der länglichen Röhre erstreckt.

12. Hüllenanordnung nach einem der vorhergehenden Ansprüche, wobei sich die echogene Struktur an einem distalen Ende (55) der Hülle und nicht an einem proximalen Ende (56) der Hülle befindet, wobei sich die echogene Struktur an dem Ende der länglichen Röhre befindet, die dem Ende gegenüberliegt, an dem sich der Verbinder befindet.

13. Hüllenanordnung nach einem der vorhergehenden Ansprüche 1-6 oder den Ansprüchen 9-12, wobei die echogene Struktur mindestens teilweise aus Metall hergestellt ist, wobei die metallische echogene Struktur keine Beschichtung ist, und wobei die metallische echogene Struktur eine Spule oder ein Geflecht umfasst.

14. Hüllenanordnung nach einem der vorhergehenden Ansprüche 1-6 oder den Ansprüchen 9-12, wobei die echogene Struktur Gasblasen im Innern der Röhrenwand der länglichen Röhre oder eine an die Röhrenwand der länglichen Röhre geklebte echogene Struktur umfasst oder wobei die echogene Struktur Partikel, insbesondere flüssige, feste oder Gelpartikel, oder mit Gas gefüllte Blasen im Innern der Wand der länglichen Röhre umfasst.

15. Kombination (200) aus einer Hüllenanordnung (10) nach einem der vorhergehenden Ansprüche 2-14 und einer Katheteranordnung oder Nadelanordnung (100), wobei eine Form des Klemmelements einem Radius der Außenseite der Kanülenwand der Katheteranordnung oder Nadelanordnung entspricht, wobei das Klemmelement entsprechend einem Krümmungsradius konkav ist.

16. Kombination nach Anspruch 15, wobei die Nadel Grate an der Außenseite oder Nuten in der Außenseite der Kanülenwand, die zum Bereitstellen von Halt für den Verbinder der Hüllenanordnung ausgelegt sind, oder eine Kanülenwand mit einem Bereich mit einer aufgerauten Oberfläche umfasst.

17. Verfahren zur Herstellung einer Hüllenanordnung nach einem der vorhergehenden Ansprüche 1-14, wobei das Verfahren umfasst:
- Bereitstellen einer länglichen Röhre mit einer echogenen Struktur,
- Verbinden der länglichen Röhre mit einem Verbinder, der dazu ausgelegt ist, an einer Außenseite einer Kanülenwand einer Nadel fixiert zu werden und dadurch die Hüllenanordnung an der Nadel zu fixieren.

## Revendications

1. Ensemble gaine (10) configuré pour être positionné sur une aiguille (102) d'un ensemble aiguille (100) ou sur une aiguille d'un ensemble cathéter, l'ensemble gaine comprenant un tube allongé (12) et un connecteur (18),
le tube allongé ayant un alésage défini (14) et comprenant une structure échogène (16), la structure échogène étant configurée pour être insérée dans le corps d'un patient et pour fournir une échogénicité à l'intérieur du corps,
**caractérisé en ce que**
le connecteur (18) est configuré pour être fixé à un extérieur de la paroi de canule de l'aiguille, fixant ainsi l'ensemble gaine.

2. Ensemble gaine selon la revendication 1, le connecteur comprenant une pince (22) configurée pour exercer une force de serrage sur l'extérieur de la paroi de canule de l'aiguille, la pince comprenant au moins un premier élément de serrage (24) et un deuxième élément de serrage (25) mobiles l'un par rapport à l'autre et configurés pour serrer l'extérieur de la paroi de canule de l'aiguille,
ou un seul élément de serrage qui a une forme annulaire et est configuré pour venir en prise circonférentiellement sur l'extérieur de la paroi de canule et une longueur circonférentielle de l'élément de serrage unique étant adaptée pour être augmentée ou diminuée afin de faire varier la force de serrage sur l'extérieur de la paroi de canule, le connecteur comprenant un élément de sollicitation (26) configuré pour exercer une force de sollicitation qui pousse les éléments de serrage l'un vers l'autre.

3. Ensemble gaine selon l'une quelconque des revendications 1 et 2, le connecteur comprenant au moins un organe de manœuvre (28) configuré pour être actionné par l'utilisateur, l'organe de manœuvre étant mobile entre une première position et une seconde position, dans la première position de l'organe de manœuvre, le connecteur n'étant pas fixé à l'aiguille, ce qui permet à l'ensemble gaine de glisser sur l'aiguille afin d'être positionné ou retiré de l'aiguille, et dans la seconde position, le connecteur étant fixé à l'aiguille.

4. Ensemble gaine selon la revendication 3, l'organe de manœuvre (28) étant configuré pour éloigner le premier et le deuxième organe de serrage l'un de l'autre contre la force de sollicitation de l'organe de sollicitation lorsque l'organe de manœuvre est déplacé vers la première position.

5. Ensemble gaine selon l'une quelconque des revendications précédentes, le connecteur n'étant pas susceptible d'être connecté à un boîtier, une enveloppe, une base, une poignée, un moyeu ou un verrouillage luer d'un ensemble aiguille.

6. Ensemble gaine selon l'une quelconque des revendications précédentes, construit et destiné à être positionné sur une aiguille d'un ensemble aiguille pour un accès vasculaire, une biopsie ou un bloc nerveux périphérique.

7. Ensemble gaine selon l'une quelconque des revendications précédentes, la structure échogène comprenant un revêtement qui est appliqué sur le tube allongé.

8. Ensemble gaine selon les revendications précédentes 1 à 6, la structure échogène comprenant un revêtement, le revêtement comprenant de petites particules ayant une impédance acoustique différente de celle de la couche de revêtement elle-même et des tissus du corps humain.

9. Ensemble gaine selon l'une quelconque des revendications précédentes, un matériau de la structure échogène ayant une impédance acoustique différente de celle des tissus humains.

10. Ensemble gaine selon l'une quelconque des revendications précédentes, la structure échogène comprenant un matériau dont la surface présente différents angles de réflexion des ondes sonores entrantes, ce qui entraîne la diffusion des ondes sonores dans toutes les directions.

11. Ensemble gaine selon l'une quelconque des revendications précédentes, la structure échogène n'étant pas appliquée sur toute la longueur du tube allongé, mais seulement sur une partie de sa longueur, la structure échogène ayant une longueur (L) qui s'étend sur au plus 50 pour cent de la longueur du tube allongé.

12. Ensemble gaine selon la revendication précédente, la structure échogène étant située à une extrémité distale (55) de la gaine et non à une extrémité proximale (56) de la gaine, la structure échogène étant située à l'extrémité du tube allongé qui est opposée à l'extrémité où se trouve le connecteur.

13. Ensemble gaine selon l'une quelconque des revendications précédentes 1 à 6 ou 9 à 12, la structure échogène étant au moins partiellement fabriquée en métal, la structure échogène métallique n'étant pas un revêtement, et la structure échogène métallique comprenant une bobine ou une tresse.

14. Ensemble gaine selon l'une quelconque des revendications précédentes 1 à 6 ou 9 à 12, la structure échogène comprenant des bulles de gaz à l'intérieur de la paroi du tube allongé ou une structure échogène collée à la paroi du tube allongé ou la structure échogène comprenant des particules, en particulier des particules liquides, solides ou de gel, ou des bulles remplies de gaz à l'intérieur de la paroi du tube allongé.

15. Combinaison (200) d'un ensemble gaine (10) selon l'une quelconque des revendications précédentes 2 à 14 et d'un ensemble cathéter ou ensemble aiguille (100), une forme de l'élément de serrage correspondant à un rayon de l'extérieur de la paroi de canule de l'ensemble cathéter ou ensemble aiguille, l'élément de serrage étant concave selon un rayon de courbure.

16. Combinaison selon la revendication 15, l'aiguille comprenant des crêtes à l'extérieur ou des rainures à l'extérieur de la paroi de canule configurées pour fournir une prise au connecteur de l'ensemble gaine ou à une paroi de canule ayant une section avec une surface rugueuse.

17. Procédé de fabrication d'un ensemble gaine selon l'une quelconque des revendications précédentes 1 à 14, le procédé comprenant :
- la fourniture d'un tube allongé ayant une structure échogène,
- la connexion dudit tube allongé à un connecteur configuré pour être fixé à un extérieur d'une paroi de canule d'une aiguille, fixant ainsi l'ensemble gaine à l'aiguille.
